# EUROPÄISCHE PATENTANMELDUNG

(11) **EP 1 774 970 A1**
(43) Veröffentlichungstag der Anmeldung: **18.04.2007**
(21) Anmeldenummer: 05024776.6
(22) Anmeldetag: 14.11.2005
(51) Int. Cl.: A61K 31/565

(54) **Verfahren zur Herstellung eines einphasigen pharmazeutischen Präparates zur Therapie der dysfunktionellen uterinen Blutung, enthaltend Ethinylestradiol und Dienogest**

(30) Priorität: 13.10.2005 EP 05022318
(71) Anmelder: Bayer Schering Pharma AG, 13353 Berlin (DE)
(72) Erfinder: Hübler, Doris, 07407 Uhlstädt-Kirchhasel (DE); Fricke, Sabine, 07749 Jena (DE)
(74) Vertreter: Cramer, Eva-Maria

(57) **Zusammenfassung**

Die Erfindung betrifft ein Verfahren zur zur Herstellung eines einphasigen pharmazeutischen Präparates zur oralen Therapie der dysfunktionellen uterinen Blutung, wobei eine orale einphasige Kombination von Dienogest und Ethinylestradiol in niedriger Dosierung verwendet wird, bei welcher durch gleich bleibende niedrige Hormonspiegel eine mäßige Proliferation des Endometriums mit vollständiger Transformation und kurzer geringer Abbruchblutung in der hormonfreien Zeit erzielt wird.

## Beschreibung

### Gebiet der Erfindung

Die Erfindung betrifft ein Verfahren zur Herstellung eines einphasigen pharmazeutischen Präparates zur oralen Therapie der dysfunktionellen uterinen Blutung, wobei eine Kombination mit gleich oder kleiner 0.020 mg Ethinylestradiol und gleich oder kleiner 2 mg 17α-Cyanomethyl-17-β-hydroxyestra-4,9-dien-3on (Dienogest), vorzugsweise 0.015 mg Ethinylestradiol und 1.5 mg DNG zu 21 Tagesdosiseinheiten verwendet wird.
Im pharmazeutischen Präparat können zusätzlich 7 oder weniger wirkstoffreie oder einnahmefreie Tagesdosiseinheiten, d.h. Tagesdosiseinheiten, die keinen Wirkstoff enthalten, umfasst sein, wobei deren Gabe im Anschluss an die Dauer von mindestens 21 aufeinander folgenden Tagen so bestimmt ist, dass die Gesamtzahl 28 Tagesdosiseinheiten oder Zyklustage beträgt.
Ferner kann das pharmazeutische Präparat auch die Kombination von Ethinylestradiol und Dienogest mit 90 bis 130, vorzugsweise 98 bis 128 Tagesdosiseinheiten umfassen.

### Allgemeiner Stand der Technik

Die dysfunktionelle uterine Blutung ist ein häufiges klinisches Problem in der Gynäkologie und wird gegenwärtig sowohl medikamentell als auch chirurgisch behandelt (Bongers MY, Mol WJB, Brölmann HAM. Current treatment of dysfunctional uterine bleeding. Maturitas 2004; 47:159-174) . Sie ist durch eine ausgiebige zyklische oder azyklische Blutung größer oder gleich 80 ml, einem Intervall zwischen den Blutungen von kleiner oder gleich 21 Tagen oder von verlängerten Blutungstagen gleich oder größer 8 Tage gekennzeichnet, jeweils aber ohne organische Ursache, wie Myome, Polypen oder Krebs. Diese Ursachen müssen vor der Diagnose - dysfunktionelle uterinen Blutung - ausgeschlossen werden.

Die dysfunktionelle uterine Blutung tritt gehäuft in Phasen der hormonellen Umstellung bei der Frau, wie Pubertät und Klimakterium auf. Durch insuffiziente Ovarialfunktion mit ausbleibender Follikulogenese und fehlender Corpus-luteum-Bildung bei zu geringer Aktivität bezüglich der ovariellen Östrogensynthese wird die dysfunktionelle uterine Blutung hervorgerufen.
Die dysfunktionelle uterine Blutung hängt häufig mit einer Anovulation zusammen. Derartige Anovulationsblutungen rekrutieren aus sich verändernden Östrogenspiegeln. Jedoch kann diese Blutung auch die Folge einer chronische Anovulation sein, welche durch unausgewogenen Östrogensekretion verursacht wird. die eine endometriale Proliferation auslöst und daher mit einem erhöhten Risiko für endometrialen Krebs verbunden ist. Es ist bekannt, dass unausgewogene Östrogenspiegel, wie überschüssiges Östrogen oder Östrogen ohne ausgewogene Gegenwirkung von Gestagenspiegeln die Ursache der dysfunktionellen uterinen Blutung sein können.

Auch die Einnahme von oralen Kontrazeptiva kann zeitweise dysfunktionelle uterine Blutungen, speziell Metrorrhagien (sehr starke und langanhaltende Blutungen) auslösen (Erny R, Erny N. Metrorrhagia caused by estrogen-progestin combinations. Contracept Fertil Sex 1994; 22(2): 93-97). Auch hier sind die Ursachen Hormonschwankungen durch vergessene Tabletten, unregelmäßige Einnahmen, ungünstige Pharmakokinetik oder nichtoptimales Ostrogen/Gestagenverhättnis.

Zur Behandlung der dysfunktionellen uterinen Blutung sind zahlreiche Therapien entwickelt worden, bei welchen Gestagene als alleinige Gabe oder eine Östrogen/Gestagen-Kombination entweder einphasig (durchgängige Gabe gleicher Dosiereung in einem bestimmten Zeitraum ) oder mehrphasig -zweiphasig oder dreiphasig - (nicht durchgängige Gabe der Ostrogen/Kombination, beispielsweise 7 Tage Östrogen-Gabe plus 14 Tage CSstrogen/Gestagen-Gabe für zweiphasig) verabreicht werden. Bei diesen verschiedenen Therapieschemata mit den unterschiedlichsten Östrogen/Gestagen-Kombinationen wurden immer hohe Hormondosierungen verwendet.

Fraser IS, Aust NZ. J Obstet Gynaecol (1990) 30(4),353-356 offenbart die Behandlung der dysfunktionellen uterinen Blutung mit einer Gabe von 5 mg Norethisteron, 3mal täglich oder 10 mg Medroxyprogesteronacetateines 3mal täglich als alleiniges hoch dosiertes Gestagen jeweils für 14 Tage vom 12. bis 25. Zyklustag.

Kaufmann, Costa und Scharl (HrsG), Die Gynäkologie, Springer Verlag Berlin Heidelberg 2003, 139 offenbaren die Behandlung der dysfunktionellen Blutung durch hoch dosierte Östrogen-Gestagen-Substitution , beispielsweise mit einem Einphasenpräparat, wie Prosiston^{®} (0,03 mg Ethinylestradiol/ 6 mg Norethisteronacetat) für 20 Tage oder die Gabe eines zweiphasigen Ovulationshemmers wie Oviol^{®} (0,05 mg Ethinylestradiol - 7 Tagesdosiseinheiten und 0,05 mg Ethinylestradiol/0,125 mg Desogestrel - 15 Tagesdosiseinheiten.
Diese hormonelle Therapie ist dabei als erste Phase einer Gesamttherapie zu betrachten, in deren Anschluss eine Substitutions- oder Simulationstherapie bzw. eine Therapie der Ursache der Ovarialstörung erfolgen sollte.

Steiner RA, Fink D. Menstruationsstörungen. Praxis 2002; 91: 1967-1974) zeigt ebenfalls auf, dass die Behandlung der dysfunktionellen uterinen Blutung u.a.mit hoch dosierten Gestagenen, Estrogenen oder Kombinationen von Beiden erfolgen sollte.
Ein Anwendungsregime sieht Steiner z.B. in der oralen Gabe von 0,01 mg Ethinylestradiol mit 2 mg Norethisteronacetat für 8 Tage in absteigender Dosierung, nämlich 6,5,4,3,3,3,3,3 Tabletten/Tag.

Davis A, Godwin A, Lippman J, Olson W, Kafrissen M. Triphasic Norgestimate-Ethinyl Estradiol for Treating Dysfunctional Uterine Bleeding. Obstet gynecol. 2000; 96(6): 913-920 zeigt die Behandlung von dysfunktioneller uteriner Blutung mittels einer dreistufigen Gabe von Ethinylestradiol (EE)/Norgestimate (NGM) gefolgt von einer hormonfreien Placebo-Gabe für drei 28-Tage Zyklen auf. Das Behandlungsregime gliedert sich dabei derart auf, dass die Dosis an EE über 21 Tage konstant bleibt (0,035 mg EE), dies Dosis an NGM über diese 21 Tage ansteigt (7 Tagesdosiseinheiten zu 0,180 mg NMG und 7 Tagesdosiseinheiten zu 0, 215 mg NMG und 7 Tagesdosiseinheiten zu 0,250 mg NMG), gefolgt von einer 7tägigen hormonfreien Placebo-Gabe.

US 6,797,282 erklärt in der Beschreibung allgemein, dass Langzeitanwendungen (3 Monate) oraler Kontrazeptiva zur Behandlung von Menorrhagien - eine Form von dysfunktioneller uteriner Blutung angewendet werden können.

### Detaillierte Offenbarung der Erfindung

Aufgabe der vorliegenden Erfindung ist es ein geeignetes Mittel bereit zu stellen, welches generell die dysfunktionelle uterine Blutung verringert, eine akute dysfunktionelle uterine Blutung stoppt, und dass Wiederauftreten einer dysfunktionellen Blutung verhindert und dabei die aufgeführten Nachteile und Wirkungender konventionellen Mittel vermeidet.

Diese Aufgabe wird erfindungsgemäß durch das Verfahren zur Herstellung eines einphasigen pharmazeutischen Präparates nach Anspruch 1 zur oralen Therapie der dysfunktionellen uterinen Blutung gelöst. Dabei enthält erfindungsgemäß das pharmazeutische Präparat eine Kombination mit gleich oder kleiner 0.020 mg Ethinylestradiol und gleich oder kleiner 2 mg 17α-Cyanomethyl-17-β-hydroxyestra-4,9-dien-3on (Dienogest), bevorzugt 0.015 mg Ethinylestradiol und 1.5 mg Dienogest zu 21 Tagesdosiseinheiten.
Die Kombination wird auch gleichzeitig zur Herstellung eines pharmazeutischen Präparates zur oralen Kontrazeption verwendet.
Ferner wird die Aufgabe durch ein pharmazeutisches Präparat nach Anspruch 5 gelöst.
Das pharmazeutische Präparat nach Anspruch 5 kann außerdem zusätzlich 7 oder weniger wirkstoffreie Tagesdosiseinheiten enthalten. Unter wirkstoffreie Tagesdosiseinheiten sind Tagesdosiseinheiten, die keinen Wirkstoff enthalten, jedoch nicht einnahmefrei sind, zu verste hen. Sie sind zur Gabe im Anschluss an die Dauer von mindestens 21 aufeinander folgenden Tagen bestimmt, so dass die Gesamtzahl an Tagesdosiseinheiten 28 beträgt.
Auch kann die Anzahl der Tagesdosiseinheiten mit der Kombination von Ethinylestradiol und Dienogest 21, 22, 23, 24, oder 25 und die wirkstoffreien Tagesdosiseinheiten 7, 6, 5, 4 oder 3 betragen.
Die Anzahl der Tagesdosiseinheiten, enthaltend die Kombination von gleich oder kleiner 0.020 mg Ethinylestradiol und gleich oder kleiner 2 mg Dienogest, bevorzugt 0.015 mg Ethinylestradiol und 1.5 mg Dienogest kann 90 bis 130, vorzugsweise 98 bis 128 Tagesdosiseinheiten umfassen.
Abhängig vom Wunsch der Frau nach kontinuierlicher Therapie der dysfunktionellen uterinen Blutung und gegebenenfalls einer ebenfalls kontinuierlichen Kontrazeption verbunden mit dem Bedürfnis nach Blutungsfreiheit über einen größeren Zeitraum kann die Anzahl der Tagesdosiseinheiten, enthaltend die Kombination von gleich oder kleiner 0-020 mg Ethinylestradiol und gleich oder kleiner 2 mg Dienogest, bevorzugt 0.015 mg Ethinylestradiol und 1.5 mg Dienogest 365 oder 366 Tagesdosiseinheiten mit einer sich anschließenden einnahmefreien Zeit umfassen.

Das erfindungsgemäß zur Minimierung von Blutungen bei dysfunktionellen uterinen Blutungen verwendete einphasige Kombionationspräparat besitzt im Vergleich zu konventionellen Präparaten eine niedrig dosierte Gesamtsteroidlast. Es ist besonders für Frauen in Phasen der hormonellen Umstellung wie Pubertät und Klimakterium geeignet, die unter anderem auch an einer hormonellen Kontrazeption interessiert sind.
Unabhängig von der Anzahl der steroidalen Tagesdosiseinheiten wird täglich ein ausgewogener Hormonspiegel realisiert, welcher eine mäßige Proliferation des Endometriums mit ausreichender vollständiger Transformation ermöglicht.
Es fördern Tagesdosiseinheiten an den verwendeten steroidalen Wirkstoffen von mindestens 21 Tagen und wirkstofffreie oder einnahmefreie Tagesdosiseinheiten von 7 Tagen und kleiner ein schnelles und vollständiges Abbluten des Endometriums. Es wird der den Zyklus stabilisiert und verhindert damit die dysfunktionelle uterine Blutung verhindert.
Ferner ermöglicht eine kontinuierliche Hormongabe mit 365/366 Tagesdosiseinheiten oder 90 bis 130 Tagesdosiseinheiten, vorzugsweise 98 bis 128, Tagesdosiseinheiten und anschließender einnahmefreier Zeit eine entsprechend längere blutungsfreie Zeit mit gleichzeitiger Hemmung der Ovulation.
Die Dauer der Gabe des pharmazeutischen Präparates umfasst mindestens drei Einnahmezyklen von 28 Tagen, wobei 21, 22, 23, 24 oder 25 Tage die steroidale Kombination nach den Ansprüchen 1 bis 3 umfasst ist und 7, 6, 5, 4 oder 3 Tagesdosiseinheiten wirkstofffrei sind.
Auch kann die Dauer der Gabe des pharmazeutischen Präparates mindestens drei Zyklen zu 28 Tagen betragen, wobei 21, 22, 23, 24 oder 25 Tage die steroidale Kombination nach den Ansprüchen 1 bis 3 umfasst und 7, 6, 5, 4 oder 3 Tagesdosiseinheiten einnahmefrei sind.

### Untersuchungen zur Wirksamkeit der beanspruchten Formulierung

In einer randomisierten, doppelt-blinden, placebo-kontrollierten, klinischen Studie werden 120 Frauen im Alter zwischen 15 und 50 Jahren mit DUB Symptomen nach schriftlicher Einverständniserklärung zur Studienteilnahme und nach Ausschluss einer organischen Ursache der Symptome mit geeigneten diagnostischen Methoden (transvaginaler Ultraschall, Bestimmung von Hormonen im Blut) behandelt.
80 Frauen erhalten Dienogest und Ethinylestradiol entsprechend der beanspruchten Kombination und 40 Frauen ein Placebo.
Der Versuch umfasste eine Run-in Phase von 90 Tagen in denen die Schwere der Blutungsstörungen aufgenommen werden, 40 Frauen erhalten 7 Behandlungszyklen zu 28 Tagen, 40 Frauen 2 Behandlungszyklen zu 98 Tagen und 40 Frauen erhalten Placebobehandlung. Alle 120 Frauen werden in einen Nachbehandlungszyklus (Follow-up-Phase) beobachtet.

Die Blutungsstärke wird durch die alkaline Hämatinmethode quantitativ erfasst. Hierzu werden die Frauen über die gesamte Studie die benutzte Monatshygiene sammeln und beim Prüfzentrum abgeben. Die Dauer der Blutung und die Dauer der blutungsfreien Intervalle werden über tägliche Dokumentation in einem elektronischen Tagebuch erfasst.

## Patentansprüche

1. Verfahren zur Herstellung eines einphasigen pharmazeutischen Präparates zur oralen Therapie der dysfunktionellen uterinen Blutung, **dadurch gekennzeichnet,**
**dass** eine Kombination mit gleich oder kleiner 0.020 mg Ethinylestradiol und gleich oder kleiner 2 mg 17α-Cyanomethyl-17-β-hydroxyestra-4,9-dien-3on (Dienogest) zu 21 Tagesdosiseinheiten verwendet wird.

2. Verfahren nach Anspruch 1, wobei die Kombination 0.015 mg Ethinylestradiol enthält.

3. Verfahren nach einem der Ansprüche 1 und 2, wobei die Kombination 1.5 mg Dienogest enthält.

4. Verfahren nach einem der Ansprüche 1 bis 3, wobei die Kombination gleichzeitig zur Herstellung eines pharmazeutischen Präparates zur oralen Kontrazeption verwendet wird.

5. Pharmazeutisches Präparat nach Anspruch 1 und 4 , enthaltend getrennt verpackte und einzeln entnehmbare Tagesdosiseinheiten, welche für die Dauer von mindestens 21 aufeinander folgenden Tagen in einer Verpackungseinheit eingebracht sind, wobei die Tagesdosiseinheiten eine Kombination mit gleich oder kleiner 0.020 mg Ethinylestradiol und gleich oder kleiner 2 mg Dienogest, bevorzugt 0.015 mg Ethinyfestradiol und 1.5 mg Dienogest enthalten.

6. Pharmazeutisches Präparat nach einem der Ansprüche 1 oder 5, wobei die Anzahl der Tagesdosiseinheiten mit der Kombination von Ethinylestradiol und Dienogest 21, 22, 23, 24, oder 25 beträgt und wobei die einnahmefreien Tagesdosiseinheiten 7, 6, 5, 4 oder 3 , so dass die Gesamtzahl an Zyklustagen 28 beträgt.

7. Pharmazeutisches Präparat nach einem der Ansprüche 1 bis 4, wobei die Kombination von Ethinylestradiol und Dienogest mit 90 bis 130, vorzugsweise 98 bis 128 Tagesdosiseinheiten umfasst ist.

8. Pharmazeutisches Präparat nach einem der Ansprüche 1 bis 4, wobei die Kombination von Ethinylestradiol und Dienogest mit 365 oder 366 Tagesdosiseinheiten umfasst ist.

9. Pharmazeutisches Präparat nach Anspruch 5 , zusätzlich enthaltend 7 oder weniger wirkstoffreie Tagesdosiseinheiten, welche zur Gabe im Anschluss an die Dauer von mindestens 21 aufeinander folgenden Tagen bestimmt sind, so dass die Gesamtzahl 28 Tagesdosiseinheiten beträgt.

10. Pharmazeutisches Präparat nach einem der Ansprüche 5 oder 9, wobei die Anzahl der Tagesdosiseinheiten mit der Kombination von Ethinylestradiol und Dienogest 21, 22, 23, 24, oder 25 beträgt und wobei die wirkstoffreien Tagesdosiseinheiten 7, 6, 5, 4 oder 3 betragen, so dass die Gesamtzahl 28 Tagesdosiseinheiten beträgt.
